(19) **Europäisches Patentamt / European Patent Office / Office européen des brevets**

(11) **EP 4 286 848 A1**

(12) # EUROPEAN PATENT APPLICATION
published in accordance with Art. 153(4) EPC

(43) Date of publication:
**06.12.2023  Bulletin 2023/49**

(21) Application number: **22746037.5**

(22) Date of filing: **28.01.2022**

(51) International Patent Classification (IPC):
*G01N 33/53* $^{(2006.01)}$       *C07K 16/18* $^{(2006.01)}$

(52) Cooperative Patent Classification (CPC):
**G01N 33/6896;** C07K 16/18; G01N 2800/2814;
G01N 2800/2821; G01N 2800/2835

(86) International application number:
**PCT/JP2022/003350**

(87) International publication number:
**WO 2022/163818 (04.08.2022 Gazette 2022/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.01.2021  JP 2021012620**

(71) Applicant: **Tohoku University
Sendai-shi, Miyagi 980-8577 (JP)**

(72) Inventors:
• **FUKUNAGA, Koji**
  **Sendai-shi, Miyagi 980-8577 (JP)**
• **KAWAHATA, Ichiro**
  **Sendai-shi, Miyagi 980-8577 (JP)**

(74) Representative: **Winter, Brandl - Partnerschaft
mbB
Alois-Steinecker-Straße 22
85354 Freising (DE)**

(54) **BIOMARKER FOR DIAGNOSIS OF DEMENTIA**

(57)    A method for detecting a neurodegenerative disease in a subject, the method including: measuring levels of two biomarkers including FABP3 and FABP5 in a biological sample from a subj ect.

**EP 4 286 848 A1**

**(Cont. next page)**

FIG. 3

**Description**

TECHNICAL FIELD

The present invention relates to a biomarker for a neurodegenerative disease and more specifically relates to a method for detecting a neurodegenerative disease using a fatty acid-binding protein (FABP) family as an index.

BACKGROUND ART

Fatty acid-binding proteins (FABPs) are involved in intracellular transportation of polyunsaturated fatty acids, such as arachidonic acid and docosahexaenoic acid. In the brain, three FABPs (FABP3, FABP5, and FABP7) are expressed. Functions of FABPs in neurodegenerative diseases have been unknown.
Inventors have recently found that FABP3 binds to $\alpha$-synuclein, which forms intracellular inclusion bodies that cause Parkinson's disease, and that co-expressing both in nerve cells forms aggregates (Non-Patent Document 1). In addition, inventors confirmed that FPBP3-deficient mice given 1-metyl-1,2,3,6-tetrahydropiridine (MPTP), a neurotoxin that causes Parkinson's disease, did not show Parkinson's disease-like symptoms and that expression and aggregation in $\alpha$-synuclein cells were suppressed in Parkinson's disease model cells and animals (Non-Patent Literature 2).
Furthermore, there is a report that serum FABP7 concentration was observed to increase in patients with a neurodegenerative disease, such as Alzheimer's dementia and Parkinson's disease (Non-Patent Literature 3).

CITATION LIST

Non-Patent Literature

Non-Patent Literature 1: Shioda N, et al. J Biol Chem. 2014 Jul 4; 289 (27): 18957-65
Non-Patent Literature 2: The Uehara Memorial Foundation Research Report Collection, 31 (2017)
Non-Patent Literature 3: Teunissen, C.E., et al. European journal of neurology 18, 865-871 (2011)

SUMMARY OF INVENTION

Technical Problem

The non-patent literatures described above suggest that FABP3 and FABP7 could serve as biomarkers for neurodegenerative diseases. However, inventors examined FABP3 concentrations in biological samples from dementia patients and found that FABP3 is observed to increase in various neurodegenerative diseases more than in healthy subjects but FABP3 alone cannot individually distinguish four neurodegenerative diseases, mild cognitive impairment (MCI), Alzheimer's disease (AD), Parkinson's disease (PD), and dementia with Lewy bodies (DLB) (MCI vs. AD N.S., MCI vs. PD N.S., AD vs. PD N.S., and PD vs. DLB N.S., where N.S. means not significantly different).
On the other hand, the association between FABP5 and neurodegenerative diseases has not been studied.

Solution to Problem

As a result of diligent studies to solve the above problems, the present inventors have found that combined use of FABP3 and FABP5 as biomarkers can distinguish whether a subject is a healthy subject or a patient likely to have a neurodegenerative disease including mild cognitive impairment and completed the present invention. Furthermore, the present inventors found that using additional one or two or more biomarkers in addition to two biomarkers FABP3 and FABP5 can individually distinguish each disease, MCI, AD, PD, and DLB.
The present invention includes embodiments described below.

Item 1. A method for detecting a neurodegenerative disease in a subject, the method including:
measuring levels of two biomarkers including FABP3 and FABP5 in a biological sample from a subject.
Item 2. The method according to item 1, the method further including:

applying and/or converting levels of the two biomarkers including FABP3 and FABP5 to a model to produce an evaluation value that is an index to detect a neurodegenerative disease; and
determining whether a subject is likely to have a neurodegenerative disease by comparing the evaluation value in the subject with a reference value.

Item 3. The method according to item 2, wherein the determining includes determining that the subject is likely to have a neurodegenerative disease when the evaluation value of the subject is higher than an evaluation value of a healthy subject.

Item 4. The method according to item 3, wherein when the subject is determined to be likely to have a mild cognitive impairment, Alzheimer's disease, Parkinson's disease, or dementia with Lewy bodies, the method further comprises:

comparing the evaluation value of the subject with each of a reference value that is an evaluation value of a patient with mild cognitive impairment, a reference value that is an evaluation value of a patient with Alzheimer's disease, a reference value that is an evaluation value of a patient with Parkinson's disease, and a reference value that is an evaluation value of a patient with dementia with Lewy bodies; and
determining that the subject is likely to have a disease with the smallest numerical difference between the evaluation value of the subject and each of the reference values of the four diseases.

Item 5. The method according to any one of items 2 to 4, wherein the model is

$$\mathrm{SCORE} = (a + Z_{\mathrm{FABP3}})/(a + Z_{\mathrm{FABP5}}) \quad (1)$$

where

$Z_{\mathrm{FABP3}}$ is (FABP3 concentration in a biological sample from a subject - FABP3 mean concentration in biological samples from healthy subjects)/(standard deviation of FABP3 concentrations in biological samples from healthy subjects),
$Z_{\mathrm{FABP5}}$ is (FABP5 concentration in a biological sample from a subject - FABP5 mean concentration in biological samples from healthy subjects)/(standard deviation of FABP5 concentrations in biological samples from healthy subjects), and
a is a number greater than zero; and

the SCORE is used for the evaluation value or the reference value.

Item 6. The method according to item 1, including further measuring a level of at least one or more biomarkers selected from the group consisting of Tau, α-synuclein, Aβ-42, GFAP, and NF-L in a biological sample from a subject.

Item 7. The method according to item 6, further including:

applying and/or converting a level of at least one or more further biomarkers selected from the group consisting of Tau, α-synuclein, Aβ-42, GFAP, and NF-L in addition to the two biomarkers FABP3 and FABP5 to a model to produce an evaluation value that is an index to detect a neurodegenerative disease; and
determining whether a subject is likely to have a neurodegenerative disease by comparing an evaluation value of the subject with a reference value that is an evaluation value of a neurodegenerative disease patient.

Item 8. The method according to item 7, wherein the neurodegenerative disease is at least one selected from the group consisting of mild cognitive impairment, Alzheimer's disease, Parkinson's disease, and dementia with Lewy bodies, and
the determining includes:

determining that the subject is likely to have mild cognitive impairment when the evaluation value of the subject is equal to or higher than a reference value that is an evaluation value of a patient with mild cognitive impairment;
determining that the subject is likely to have Alzheimer's disease when the evaluation value of the subject is equal to or higher than a reference value that is an evaluation value of a patient with Alzheimer's disease;
determining that the subject is likely to have Parkinson's disease when the evaluation value of the subject is equal to or higher than a reference value that is an evaluation value of a patient with Parkinson's disease; or
determining that the subject is likely to have dementia with Lewy bodies when the evaluation value of the subject is equal to or higher than a reference value that is an evaluation value of a patient with dementia with Lewy bodies.

Item 9. The method according to item 8, wherein when the subject is determined to be likely to have mild cognitive impairment, Alzheimer's disease, Parkinson's disease, or dementia with Lewy bodies described above, the method further includes:

comparing the evaluation value of the subject with each of a reference value that is an evaluation value of a patient with mild cognitive impairment, a reference value that is an evaluation value of a patient with Alzheimer's disease, a reference value that is an evaluation value of a patient with Parkinson's disease, and a reference value that is an evaluation value of a patient with dementia with Lewy bodies; and

determining that the subject is likely to have a disease with the smallest numerical difference between the evaluation value of the subject and each of the reference values of the four diseases.

Item 10. The method according to any one of items 7 to 9, wherein the model is

$$\text{SCORE} = (a + Z_{\text{FABP3}})(a + Z_{\text{Tau}})^x (a + Z_{\text{NF-L}})^y (a + Z_{\text{GFAP}})^z /$$
$$\{(a + Z_{\text{FABP5}}) \times (a + Z_{\text{a-syn}})^m \times (a + Z_{\text{A}\beta\text{-42}})^n\} \times a^{(m+n-x-y-z)} \quad (2)$$

where

$Z_{\text{FABP3}}$ is (FABP3 concentration in a biological sample from a subject - FABP3 mean concentration in biological samples from healthy subjects)/(standard deviation of FABP3 concentrations in biological samples from healthy subjects),

$Z_{\text{Tau}}$ is (Tau concentration in a biological sample from a subject - Tau mean concentration in biological samples from healthy subjects)/(standard deviation of Tau concentrations in biological samples from healthy subjects),

$Z_{\text{NF-L}}$ is (NF-L concentration in a biological sample from a subject - NF-L mean concentration in biological samples from healthy subjects)/(standard deviation of NF-L concentrations in biological samples from healthy subjects),

$Z_{\text{GFAP}}$ is (GFAP concentration in a biological sample from a subject - GFAP mean concentration in biological samples from healthy subjects)/(standard deviation of GFAP concentrations in biological samples from healthy subjects),

$Z_{\text{FABP5}}$ is (FABP5 concentration in a biological sample from a subject - FABP5 mean concentration in biological samples from healthy subjects)/(standard deviation of FABP5 concentrations in biological samples from healthy subjects), and

$Z_{\alpha\text{-syn}}$ is ($\alpha$-synuclein concentration in a biological sample from a subject - $\alpha$-synuclein mean concentration in biological samples from healthy subjects)/(standard deviation of $\alpha$-synuclein concentrations in biological samples from healthy subjects),

$Z_{\text{A}\beta\text{-42}}$ is (A$\beta$-42 concentration in a biological sample from a subject - A$\beta$-42 mean concentration in biological samples from healthy subjects)/(standard deviation of A$\beta$-42 concentrations in biological samples from healthy subjects),

a is a number greater than zero, and

x, y, z, m, and n are independently 1 when evaluation values of biomarkers Tau, GFAP, $\alpha$-synuclein, A$\beta$-42, and NF-L associated at bases of these multipliers in Equation (2) are calculated, or 0 when the evaluation values are not calculated; and

the SCORE is used for the evaluation value or the reference value.

Item 11. Use of a combination of two including FABP3 and FABP5;

a combination of four including FABP3, FABP5, Tau, and NF-L;
a combination of four including FABP3, FABP5, $\alpha$-synuclein, and A$\beta$-42;
a combination of five including FABP3, FABP5, $\alpha$-synuclein, A$\beta$-42, and GFAP; or
a combination of six including FABP3, FABP5, $\alpha$-synuclein, A$\beta$-42, GFAP, and NF-L

as biomarkers to examine at least one neurodegenerative disease selected from the group consisting of mild cognitive impairment, Alzheimer's disease, Parkinson's disease, and dementia with Lewy bodies.

Item 12. A kit for examination to examine at least one neurodegenerative disease selected from the group consisting of mild cognitive impairment, Alzheimer's disease, Parkinson's disease, and dementia with Lewy bodies, the kit including:

antibodies against each of two proteins including FABP3 and FABP5;
antibodies against each of four proteins including FABP3, FABP5, Tau, and NF-L;
antibodies against each of four proteins including FABP3, FABP5, $\alpha$-synuclein, and A$\beta$-42;
antibodies against each of five proteins including FABP3, FABP5, $\alpha$-synuclein, A$\beta$-42, and GFAP; or

antibodies against each of six proteins including FABP3, FABP5, $\alpha$-synuclein, A$\beta$-42, GFAP, and NF-L.

Advantageous Effects of Invention

According to the present invention, a subject can be examined for the likelihood of having a neurodegenerative disease, especially mild cognitive impairment. Furthermore, using additional one or two or more biomarkers in addition to two biomarkers FABP3 and FABP5 enables individual examination or individualized diagnosis of MCI, AD, PD, and DLB.

BRIEF DESCRIPTION OF DRAWINGS

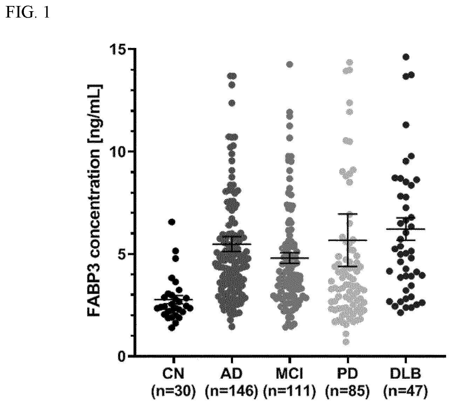

FIG. 1 is a graph comparing SCOREs of a control group and each neurodegenerative disease group when FABP3 was used as a marker. CN, healthy control; MCI, mild cognitive impairment; AD, Alzheimer's disease; and PD, Parkinson's disease. SCOREs of the four groups were all not significantly different from that of CN (Welch's t-test).

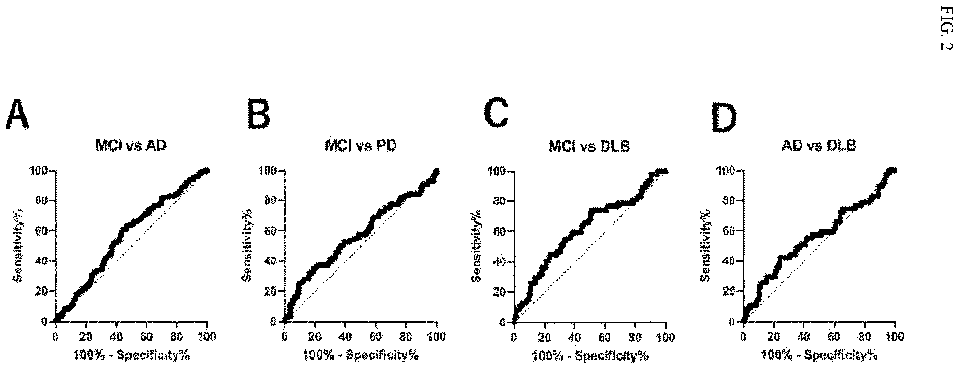

FIGS. 2 (A) to (D) are graphs comparing ROC curves of various two groups when FABP3 was used as a marker. Vertical axis, sensitivity; and horizontal axis, false-positive rate (1 - specificity). CN, healthy control; MCI, mild cognitive impairment; AD, Alzheimer's disease; PD, Parkinson's disease; and DLB, dementia with Lewy bodies.

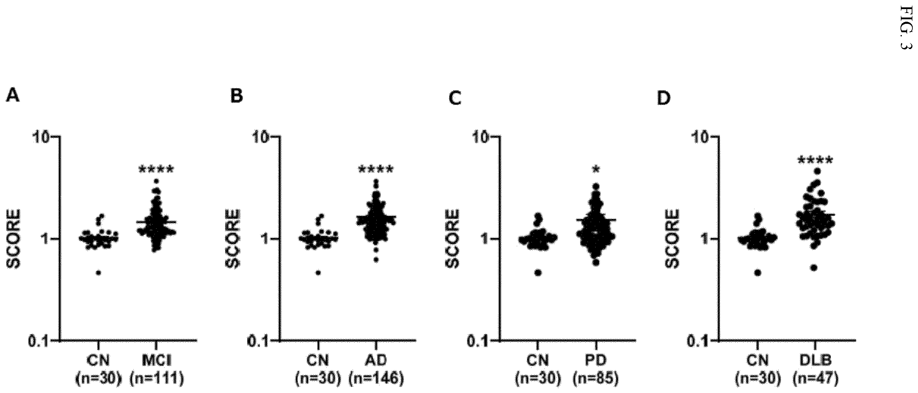

FIGS. 3 (A) to (D) are graphs comparing SCOREs of a control group and each neurodegenerative disease group when FABP3 and FABP5 were used as markers. CN, healthy control; MCI, mild cognitive impairment; AD, Alzheimer's disease; PD, Parkinson's disease; and DLB, dementia with Lewy bodies. * p < 0.05, and **** p < 0.0001; all in comparison with CN (Welch's t-test).

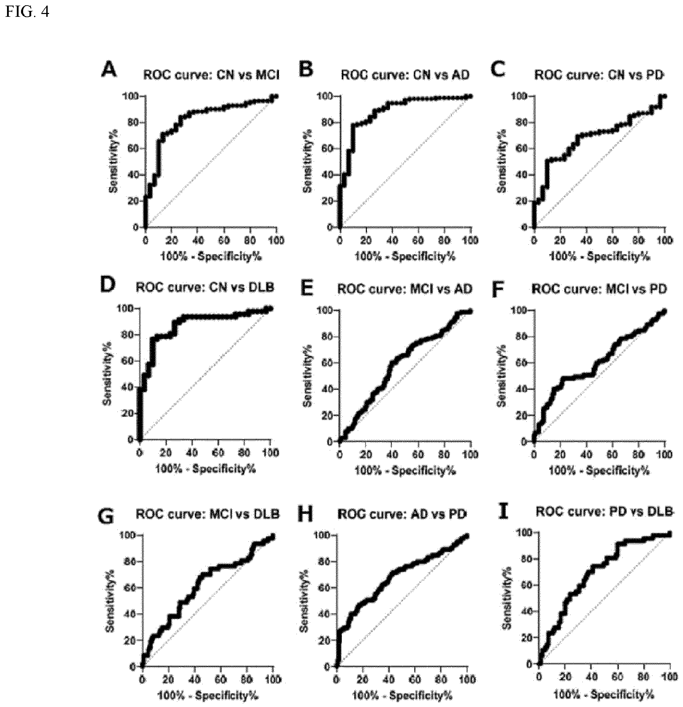

FIGS. 4 (A) to (F) are graphs comparing ROC curves of various two groups when FABP3 and FABP5 were used as markers. Vertical axis, sensitivity; horizontal axis, false-positive rate (1 - specificity). CN, healthy control; MCI, mild cognitive impairment; AD, Alzheimer's disease; PD, Parkinson's disease; and DLB, dementia with Lewy bodies.

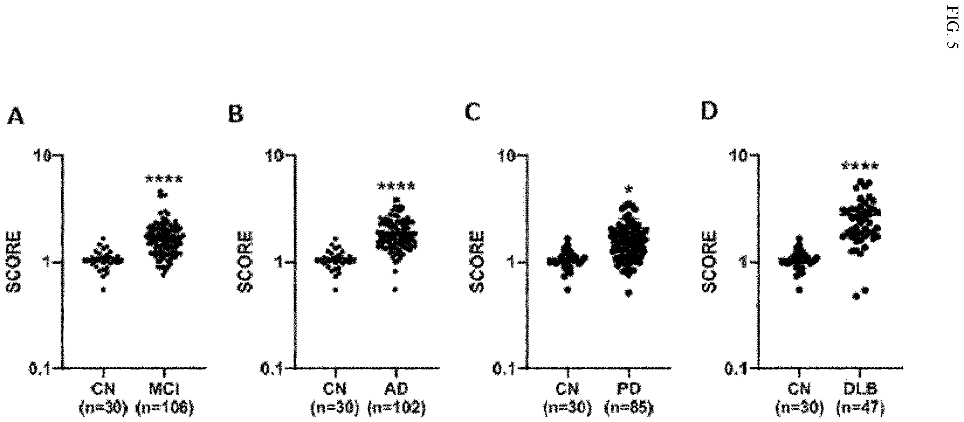

FIGS. 5 (A) to (D) are graphs comparing SCOREs of a control group and each neurodegenerative disease group when FABP3, FABP5, $\alpha$-synuclein, and A$\beta$-42 were used as markers. CN, healthy control; MCI, mild cognitive impairment; AD, Alzheimer's disease; PD, Parkinson's disease; and DLB, dementia with Lewy bodies. * p < 0.05, and **** p < 0.0001; all in comparison with CN (Welch's t-test).

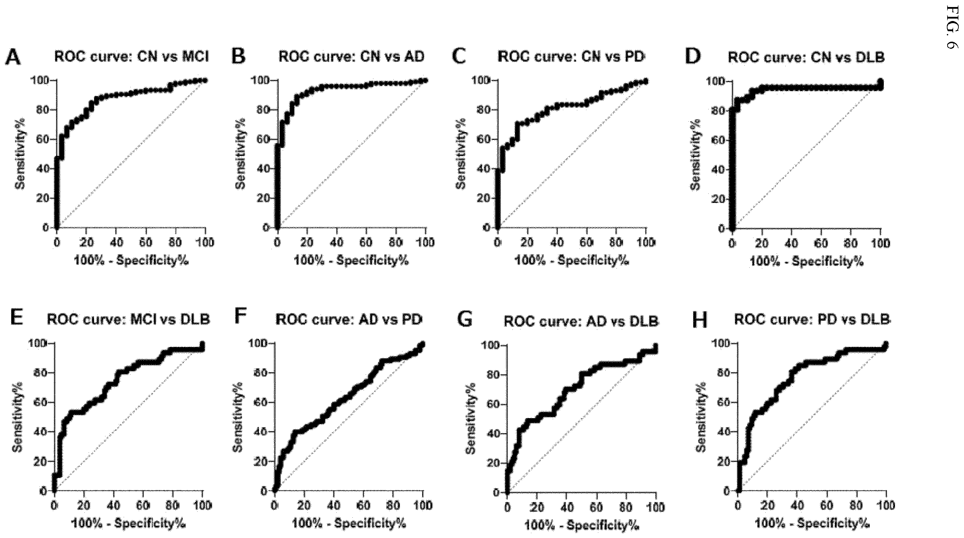

FIGS. 6 (A) to (H) are graphs comparing ROC curves of various two groups when FABP3, FABP5, $\alpha$-synuclein, and A$\beta$-42 were used as markers. Vertical axis, sensitivity; horizontal axis, false-positive rate (1 - specificity). CN, healthy control; MCI, mild cognitive impairment; AD, Alzheimer's disease; PD, Parkinson's disease; and DLB, dementia with Lewy bodies.

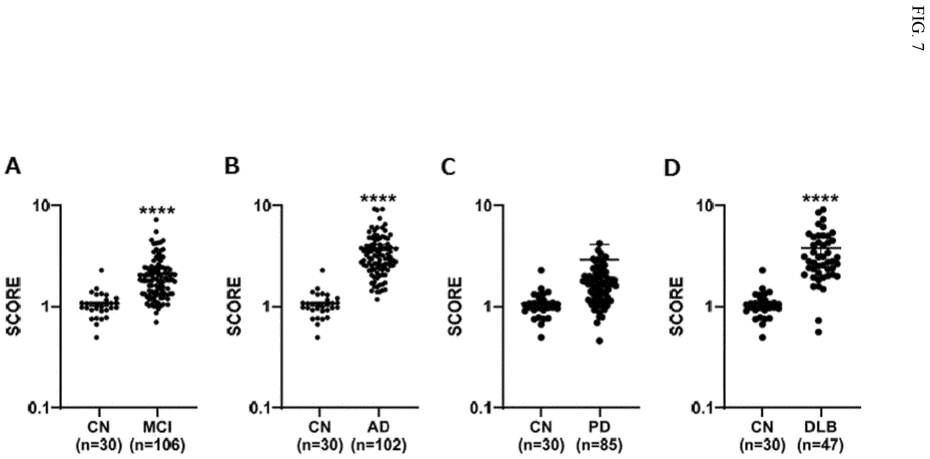

FIGS. 7 (A) to (D) are graphs comparing SCOREs of a control group and each neurodegenerative disease group when FABP3, FABP5, $\alpha$-synuclein, A$\beta$-42, and GFAP were used as markers. CN, a healthy control group; MCI, mild cognitive impairment; AD, Alzheimer's disease; PD, Parkinson's disease; and DLB, dementia with Lewy bodies. **** p < 0.0001; all in comparison with CN (Welch's t-test).

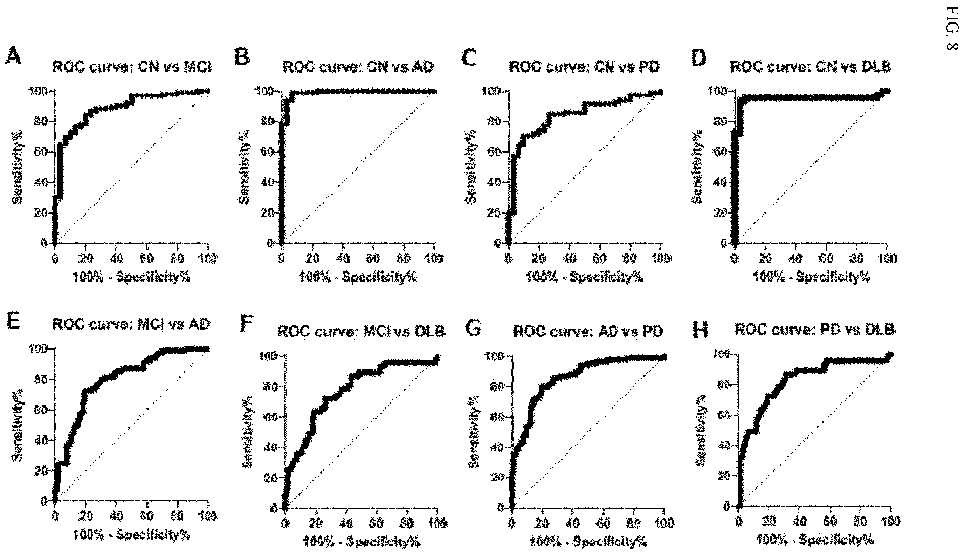

FIGS. 8 (A) to (H)) are graphs comparing ROC curves of various two groups when FABP3, FABP5, $\alpha$-synuclein, A$\beta$-42, and GFAP were used as markers. Vertical axis, sensitivity; horizontal axis, false-positive rate (1 - specificity). CN, a healthy control group; MCI, mild cognitive impairment; AD, Alzheimer's disease; PD, Parkinson's disease; and DLB, dementia with Lewy bodies.

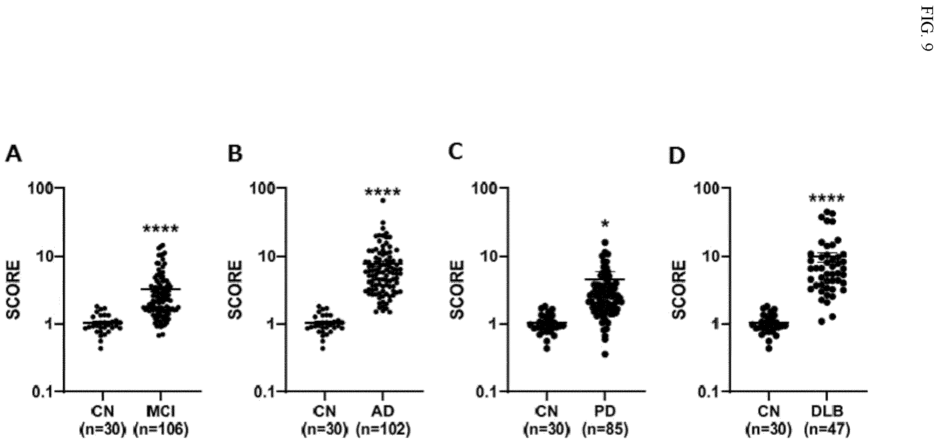

FIGS. 9 (A) to (D) are graphs comparing SCOREs of a control group and each neurodegenerative disease group when FABP3, FABP5, $\alpha$-synuclein, A$\beta$-42, GFAP, and NF-L were used as markers. CN, a healthy control group; MCI, mild cognitive impairment; AD, Alzheimer's disease; PD, Parkinson's disease; and DLB, dementia with Lewy bodies. * p < 0.05, and **** p < 0.0001; all in comparison with CN (Welch's t-test).

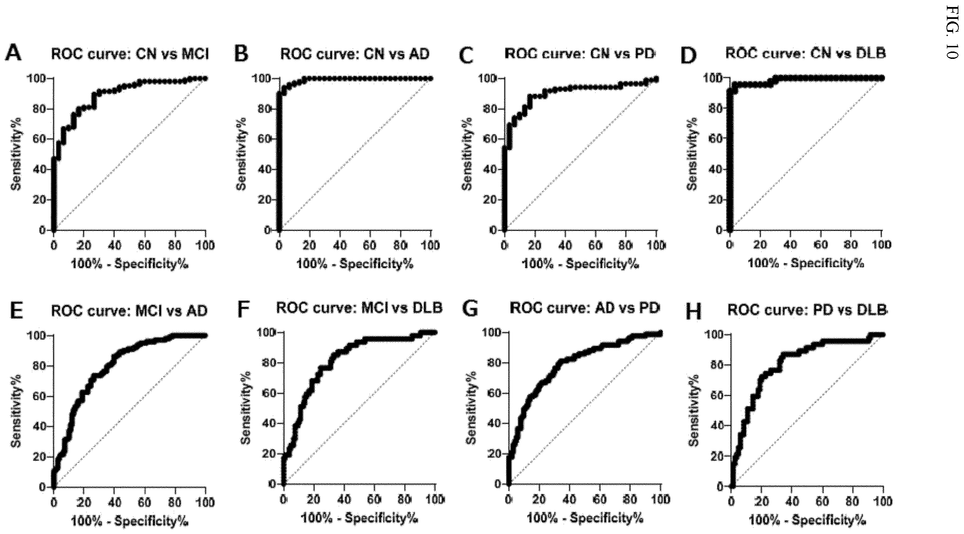

FIGS. 10 (A) to (H)) are graphs comparing ROC curves of various two groups when FABP3, FABP5, $\alpha$-synuclein, A$\beta$-42, GFAP, and NF-L were used as markers. Vertical axis, sensitivity; horizontal axis, false-positive rate (1 - specificity). CN, a healthy control group; MCI, mild cognitive impairment; AD, Alzheimer's disease; PD, Parkinson's disease; and DLB, dementia with Lewy bodies.

DESCRIPTION OF EMBODIMENTS

In the present specification, a "subject" is a mammal including a human, a mouse, and a rat; further a farm animal, such as cattle and a horse; and a pet, such as a dog and a cat; and preferably a human.

In the present specification, for a "biological sample" from a subject, a biological sample derived from a subject is not particularly limited but is preferably a sample that can be collected less invasively from the subject, and examples include those that can be easily collected from a living organism, such as blood, plasma, serum, saliva, urine, tear fluid, and sweat; those that can be relatively easily collected, such as spinal fluid, bone marrow fluid, pleural effusion, ascites, synovial fluid, aqueous humor, vitreous humor, and lymph; and tissue samples, such as those of tissue biopsy. In some embodiments, the biological sample contains a body fluid selected from the group consisting of blood (whole blood), plasma, serum, saliva, urine, bone marrow fluid, pleural effusion, ascites, synovial fluid, tear fluid, sweat, aqueous humor, vitreous humor, and lymph. When blood or serum is used, blood is collected from a subject according to a common procedure, and a sample to be analyzed can be prepared directly without pretreatment or by separating a liquid component. From a peptide of the present invention to be detected, a high molecular weight protein fraction or the like can be separated and removed in advance as necessary using an antibody column or another adsorbent column, a spin column, or the like.

A neurodegenerative disease in the present specification includes at least one selected from the group consisting of mild cognitive impairment (MCI), Alzheimer's disease (AD), Parkinson's disease (PD), and dementia with Lewy bodies (DLB). In a preferred embodiment, the neurodegenerative disease is at least one selected from the group consisting of mild cognitive impairment (MCI), Alzheimer's disease (AD), Parkinson's disease (PD), and dementia with Lewy bodies (DLB).

In the present specification, "detection" of a neurodegenerative disease includes determination of a neurodegenerative disease, determination (companion diagnostic method) of a neurodegenerative disease patient (responder) for whom a therapeutic drug is effective, determination of a preventive effect on a neurodegenerative disease, determination of a therapeutic effect on a neurodegenerative disease, an examination method for diagnosis (especially early diagnosis) of a neurodegenerative disease, and an examination method for treatment (especially early treatment) of a neurodegenerative disease. The "determination" of a neurodegenerative disease includes not only determining the presence or absence of a neurodegenerative disease but also preventively determining the likelihood of having a neurodegenerative disease, predicting the prognosis of a neurodegenerative disease after treatment, and determining the therapeutic efficacy of a therapeutic drug for a neurodegenerative disease. A method for screening a substance includes a method for screening a substance useful for the "detection", "determination", and "treatment" of a neurodegenerative disease.

In the present specification, "having a disease" includes "onset of a disease".

In the present specification, "treatment" means a cure or amelioration of a disease or symptom, or suppression of a symptom, and includes "prevention". "Prevention" means forestalling the onset of a disease or symptom.

In the present specification, FABP3 refers to fatty acid-binding protein 3. For example, in humans, FABP3 includes the protein with Uniprot accession number P05413. In mice, FABP3 includes the protein with Uniprot accession number P11404. In rats, FABP3 includes the protein with Uniprot accession number P07483.

In the present specification, FABP5 refers to fatty acid-binding protein 5. For example, in humans, FABP5 includes the protein with Uniprot accession number Q01469. In mice, FABP5 includes the protein with Uniprot accession number Q05816. In rats, FABP5 includes the protein with Uniprot accession number P55053.

In an aspect of the present invention, a method for detecting a neurodegenerative disease in a subject is provided, the method including measuring levels of two biomarkers including FABP3 and FABP5 in a biological sample from a subject.

The subject includes a patient suspected of having a neurodegenerative disease, and "a patient suspected of having a neurodegenerative disease" may be a subject who has subjective suspicions themselves that they have a neurodegenerative disease (not limited to those having subjective symptoms but including those who merely want to undergo preventive examination) or those who are determined or diagnosed as having a neurodegenerative disease based on any objective evidence (e.g., one determined by a medical doctor to have a reasonable likelihood of having a neurodegenerative disease as a result of a cognitive function test known in the art (e.g., the mini-mental state examination (MMSE) or the Hasegawa Dementia Scale (HDS-R)) and/or medical examination).

"Measuring levels of two biomarkers including FABP3 and FABP5" refers to measuring concentration, amount, or signal intensity of each of proteins FABP3 and FABP5. Measuring intensities of fluorescence signals or absorption signals of FABP3 and FABP5 enables not only qualitative measurements but also quantitative measurements to be performed.

Examples of the detection of two biomarker proteins in the biological sample include, but are not limited to, mass spectroscopy, protein array technology (e.g., a protein chip), gel electrophoresis, and methods using antibodies against each of two biomarker proteins (e.g., immunofluorescence, radioactive labeling, immunohistochemistry, immunoprecipitation, Western blot analysis, enzyme-linked immunosorbent assay (ELISA), and fluorescent cell sorting (such as FACS, immunoblotting, and chemiluminescence).

Antibodies against each of FABP3 and FABP5 can be produced by a method well known in the art as epitopes, which are partial amino acid regions (antigenic determinants) having antigenicity or immunogenicity in each protein, and commercially available antibodies against each of FABP3 and FABP5 may be used. The antibody or its parent protein of the present invention (which may be hereinafter referred to as the "antibody of the present invention") may be either a polyclonal antibody or a monoclonal antibody and can be produced by a well-known immunological technique. In addition, the antibody includes not only a complete antibody molecule but also its fragment, and examples include Fab, F(ab')2, ScFv, and a minibody.

In some embodiments, the detection/quantification of a biomarker in a biological sample can be achieved using enzyme-linked immunosorbent assay (ELISA). ELISA can be based, for example, on colorimetry, chemiluminescence, and/or fluorescence. In ELISA to be suitably used in the method of the present invention, any appropriate capture reagent and detection reagent containing an antibody and its derivative, as well as a protein ligand, and the like can be used.

In some embodiments, the detection/quantification of a biomarker in a biological sample can be achieved using Western blotting. Western blotting is well known to those skilled in the art. To describe it briefly, an antibody with binding affinity for a given biomarker is used, and the biomarker can be quantified in a mixture of proteins separated on the basis of size by gel electrophoresis. For example, a membrane made of nitrocellulose or poly(vinylidene fluoride) (PVDF is placed next to a gel containing a protein mixture from a biological sample, and an electric current is applied to transfer proteins from the gel to the membrane. The membrane is then brought into contact with an antibody with specificity for the biomarker of interest, and the biomarker can be visualized using a secondary antibody and/or a detection reagent.

In some embodiments, the detection/quantification of a biomarker in a biological sample can be achieved using a multiplex protein assay (e.g., a planar assay or a bead-based assay). Many multiplex protein assay formats are commercially available.

In other embodiments, the detection/quantification of a biomarker in a biological sample can be achieved by flow cytometry, which is a technique for counting, examining, and sorting a target entity (e.g., a cell and protein) suspended in a fluid stream.

In some embodiments, the detection/quantification of a biomarkers in a biological sample can be achieved by immunohistochemistry or immunocytochemistry, which is a process of localizing a protein to a tissue section or cell using an antibody or a protein binding agent with binding specificity for an antigen in a tissue or cell. The biomarker can be visualized by tagging the antibody/agent with a label that produces color (e.g., horseradish peroxidase and alkaline phosphatase) or fluorescence (e.g., fluorescein isothiocyanate (FITC) or phycoerythrin (PE)).

FABP3 tends to increase in patients with a neurodegenerative disease more than in healthy subjects, and FABP5 tends to decrease in patients with a neurodegenerative disease more than in healthy subjects. However, FABP3 and FABP5 have low detection accuracy when each is used alone as a biomarker.

According to the method of the present invention, a healthy subject and a patient with a neurodegenerative disease can be distinguished using two biomarkers including FABP3 and FABP5. More specifically, this method can determine whether a subject is likely to have mild cognitive impairment, Alzheimer's disease, Parkinson's disease, or dementia with Lewy bodies. Thus, this method can easily distinguish whether a subject may have mild cognitive impairment.

In some embodiments, based on an evaluation value that is an index to detect a neurodegenerative disease described later and calculated using two biomarkers including FABP3 and FABP5, an evaluation value of a subject is compared with a reference value that is an evaluation value of a healthy subject, and when the evaluation value of the subject is equal to or lower than the reference value that is an evaluation value of a healthy subject, the subject can be determined to be less likely to have a neurodegenerative disease and thus to be less likely to have mild cognitive impairment either.

In another embodiment, based on an evaluation value that is an index to detect a neurodegenerative disease described later and calculated using two biomarkers including FABP3 and FABP5, an evaluation value of a subject is compared with a reference value that is an evaluation value of a healthy subject, and when the evaluation value of the subject is greater than the reference value that is an evaluation value of a healthy subject, the subject can be determined to be likely to have a neurodegenerative disease (mild cognitive impairment, Alzheimer's disease, Parkinson's disease, dementia with Lewy bodies, or a combination of these).

In another embodiment, an evaluation value of a subject is compared with a reference value that is an evaluation value of a patient with a neurodegenerative disease (mild cognitive impairment, Alzheimer's disease, Parkinson's disease, dementia with Lewy bodies, or a combination of these), and when the evaluation value of the subject is equal to or greater than the reference value that is an evaluation value of a neurodegenerative disease patient, the subject can be determined to be likely to have a neurodegenerative disease and thus it is possible they have a mild cognitive impairment.

In a further embodiment, an evaluation value of a subject is compared with a reference value that is an evaluation value of a patient with mild cognitive impairment, Alzheimer's disease, Parkinson's disease, or dementia with Lewy bodies, and when the evaluation value of the subject is equal to or greater than the reference value that is an evaluation value of a patient with any of the four neurodegenerative diseases, the subject can be determined to be likely to have the neurodegenerative disease. The likelihood of having an individual neurodegenerative disease, mild cognitive impairment, Alzheimer's disease, Parkinson's disease, and dementia with Lewy bodies, can thus also be determined individually.

In yet another embodiment, when a subject is determined to be likely to have mild cognitive impairment, Alzheimer's disease, Parkinson's disease, or dementia with Lewy bodies, the method of the present invention further includes:

comparing the evaluation value of the subject with each of a reference value that is an evaluation value of a patient with mild cognitive impairment, a reference value that is an evaluation value of a patient with Alzheimer's disease, a reference value that is an evaluation value of a patient with Parkinson's disease, and a reference value that is an evaluation value of a patient with dementia with Lewy bodies; and

determining that the subject is likely to have a disease with the smallest numerical difference between the evaluation value of the subject and each of the reference values of the four diseases. For example, when the evaluation value of a subject has the smallest difference from a reference value that is an evaluation value of a patient with mild cognitive impairment among reference values of the four diseases, the subject can be determined to be likely to have mild cognitive impairment.

In the present specification, the evaluation value of a healthy subject is any value that serves as an index indicating that a subject is a healthy subject and not particularly limited and may be a mean value of evaluation values of multiple healthy subjects, a median of evaluation values of multiple healthy subjects, or the like, and is preferably a mean value of evaluation values of multiple healthy subjects. The evaluation value of a patient with a neurodegenerative disease (mild cognitive impairment, Alzheimer's disease, or dementia with Lewy bodies) is any value that serves as an index indicating that a subject is a patient with a neurodegenerative disease and not particularly limited and may be a mean value of evaluation values of multiple neurodegenerative disease patients, a median of evaluation values of multiple neurodegenerative disease patients, or the like, and is preferably a mean value of evaluation values of multiple neurodegenerative disease patients. For example, the evaluation value of a patient with mild cognitive impairment is any value that serves as an index indicating that a subject is a patient with mild cognitive impairment and not particularly limited and may be a mean value of evaluation values of multiple mild cognitive impairments, a median of evaluation values of multiple mild cognitive impairments, or the like, and the evaluation value of a patient with Alzheimer's disease, the evaluation value of a patient with Parkinson's disease, and the evaluation value of a patients with dementia with Lewy bodies are also similarly defined.

In some embodiments, the method for detecting a neurodegenerative disease in a subject of the present invention includes:

applying and/or converting levels of two biomarkers including FABP3 and FABP5 to a model to produce an evaluation value that is an index to detect a neurodegenerative disease; and

determining whether a subject is likely to have a neurodegenerative disease by comparing the evaluation value in the subject with a reference value.

The reference value can be an evaluation value of a healthy subject, an evaluation value of a patient with mild cognitive impairment, an evaluation value of a patient with Alzheimer's disease, an evaluation value of a patient with Parkinson's disease, and an evaluation value of a patient with dementia with Lewy bodies. The method of determination is as described above.

The model refers to a mathematical expression and preferably is an equation containing a fraction where a sum of one of FABP3 and FABP5 and a constant is a denominator, and a sum of the other of FABP3 and FABP5 and the constant is a numerator. The conversion refers to conversion of a numerical value of a level of the biomarker, and examples include use of an exponential function, a power function, and/or a root function.

One example is a model represented by Equation (1) below to distinguish a subject, which has been developed by the present inventors:

$$\text{SCORE} = (a + Z_{FABP3})/(a + Z_{FABP5}) \ (1)$$

where

$Z_{FABP3}$ is (FABP3 concentration in a biological sample from a subject - FABP3 mean concentration in biological samples from healthy subjects)/(standard deviation of FABP3 concentrations in biological samples from healthy subjects),

$Z_{FABP5}$ is (FABP5 concentration in a biological sample from a subject - FABP5 mean concentration in biological samples from healthy subjects)/(standard deviation of FABP5 concentrations in biological samples from healthy subjects), and

a is a number greater than zero, that is, a positive number, preferably a positive integer or a positive decimal, more preferably a number ranging from 1 to 10, and for example, a can be any integer from 1 to 10.

The above SCORE is used for the evaluation value or reference value that is an index to detect a neurodegenerative disease.

In Equation (1), the SCORE value is 1 in healthy subjects, but the mean value of SCORE is greater than 1 in all patient groups with mild cognitive impairment, Alzheimer's disease, Parkinson's disease, or dementia with Lewy bodies. When the levels of two biomarkers FABP3 and FABP5 are substituted into Equation (1), SCORE values are significantly different respectively between the mean value of a healthy subject group and the mean value of a patient group with mild cognitive impairment, between the mean value of a healthy subject group and the mean value of a patient group with Alzheimer's disease, between the mean values of healthy subjects and a Parkinson's disease patient group, between the mean value of a healthy subject group and the mean value of Lewy body disease, between the mean value of a patient group with mild cognitive impairment and the mean value of a patient group with Alzheimer's disease, between the mean value of a patient group with mild cognitive impairment and the mean value of a Parkinson's disease patient group, between the mean value of a patient group with mild cognitive impairment and the mean value of a patient group with Lewy body disease, between the mean value of a patient group with Alzheimer's disease and the mean value of a Parkinson's disease patient group, and between the mean value of a Parkinson's disease patient group and the mean value of a patient group with Lewy body disease, and the latter is significantly greater than the former, respectively.

Thus, whether a subject has any of these neurodegenerative diseases can be determined using the SCORE value obtained by Equation (1) as an index.

For example, a SCORE value of Equation (1) of a subject is compared with a reference value that is a mean value of SCORE values of healthy subjects, that is, 1, and when the SCORE value of the subject is equal to or lower than 1, the subject can be determined to be less likely to have a neurodegenerative disease and thus to be less likely to have mild cognitive impairment either.

A SCORE value of Equation (1) of a subject is compared with a reference value that is a mean value of SCORE values of patients with a neurodegenerative disease (mild cognitive impairment, Alzheimer's disease, Parkinson's disease, dementia with Lewy bodies, or a combination of these), and when the SCORE value of the subject is equal to or greater than the reference value that is a mean value of SCORE values of neurodegenerative disease patients, the subject can be determined to be likely to have a neurodegenerative disease and thus to be likely to have mild cognitive impairment.

In some embodiments, a SCORE value of Equation (1) of a subject is compared with a reference value that is a mean value of SCORE values of patients with mild cognitive impairment, Alzheimer's disease, Parkinson's disease, or dementia with Lewy bodies, and the likelihood of having an individual neurodegenerative disease, mild cognitive impairment, Alzheimer's disease, Parkinson's disease, and dementia with Lewy bodies, can also be determined individually. For example, when the SCORE value of a subject is equal to or higher than a reference value that is a SCORE value of a patient with mild cognitive impairment, the subject can be determined to be likely to have mild cognitive impairment. When the SCORE value of a subject is equal to or higher than a reference value that is a SCORE value of a patient with Alzheimer's disease, the subject can be determined to be likely to have Alzheimer's disease. When the SCORE value of a subject is equal to or higher than a reference value that is a SCORE value of a patient with Parkinson's disease, the subject can be determined to be likely to have Parkinson's disease. When the SCORE value of a subject is equal to or higher than a reference value that is a SCORE value of a patient with dementia with Lewy bodies, the subject can be determined to be likely to have dementia with Lewy bodies.

In yet another embodiment, when a subject is determined to be likely to have mild cognitive impairment, Alzheimer's disease, Parkinson's disease, or dementia with Lewy bodies, the method of the present invention further includes:

comparing the SCORE value of Equation (1) of the subject with each of a reference value that is a SCORE value of a patient with mild cognitive impairment, a reference value that is a SCORE value of a patient with Alzheimer's disease, a reference value that is a SCORE value of a patient with Parkinson's disease, and a reference value that is a SCORE value of a patient with dementia with Lewy bodies; and
determining that the subject is likely to have a disease with the smallest numerical difference between the SCORE value of the subject and each of the reference values of the four diseases.

In the present application, a multivariate model represented by Equation (1) of biomarker proteins, Equation (2) described later, and Equation (3) described later has been constructed, and this has been found to enable very highly reliable detection or determination of a neurodegenerative disease with high area under the curve (AUC) (exceeding 0.8) of the receiver operating characteristic (ROC) analysis. The ROC analysis is a non-limiting example of analysis and may include comparing true classification based on criteria of an appropriate reference substance and classification of each test patient. Classification of multiple patients by such a method may allow the derivation of measurements of sensitivity and specificity. Sensitivity is commonly a proportion of correctly classified patients among all true positive patients, and specificity is a proportion of correctly classified cases among all true negative cases. A trade-off may commonly exist between sensitivity and specificity depending on the threshold selected to determine positive classification. A low threshold commonly has high sensitivity but may have relatively low specificity. In contrast, a high threshold commonly has low sensitivity but may have relatively high specificity. The ROC curve can be constructed by horizontally inverting a plot of sensitivity versus specificity. The resulting inverted horizontal axis is a false-positive fraction and equal to specificity subtracted from 1. The area under the ROC curve (AUC) can be interpreted as a mean sensitivity over the entire range of possible specificities or a mean specificity over the entire range of possible sensitivities. The AUC represents a measure of overall accuracy and also represents a measure of accuracy covering all possible interpretation thresholds.

Increasing the number of biomarker proteins increases the AUC to 0.9 or greater, and this may allow the AUC to be closer to 1. Even if the AUC is 0.8 or less with one biomarker, the biomarker is effective as a biomarker for detecting or determining a neurodegenerative disease as long as it can be used to detect or determine a neurodegenerative disease with an AUC exceeding 0.8 and preferably exceeding 0.9, for example, by combining multiple biomarker proteins.

In addition to FABP3 and FABP5, one or two or more biomarkers may be used in the method for detecting a neurodegenerative disease in a subject of the present invention. In general, using a greater number of biomarkers can further increase the sensitivity (true positive rate) and specificity (true negative rate) and increases the detection accuracy.

In some embodiments, the method for detecting a neurodegenerative disease in a subject of the present invention further includes:

applying and/or converting a level of at least one or more further biomarkers selected from the group consisting of Tau, NF-L, α-synuclein, Aβ-42, GFAP, and NF-L in addition to two biomarkers FABP3 and FABP5 to a model to produce an evaluation value that is an index to detect a neurodegenerative disease; and

determining whether the subject is likely to have a neurodegenerative disease by comparing the evaluation value of the subject with a reference value.

The reference value can be an evaluation value of a healthy subject, an evaluation value of a patient with mild cognitive impairment, an evaluation value of a patient with Alzheimer's disease, an evaluation value of a patient with Parkinson's disease, and an evaluation value of a patient with dementia with Lewy bodies. The method of determination is as described above. Microtubule-associated protein tau (Tau, Uniprot accession number: P10636), alpha-synuclein (α-Synuclein, Uniprot accession number: P37840), amyloid beta protein (Aβ-42, Uniprot accession number: Q9BYY9), glial fibrillary acidic protein (GFAP, Uniprot accession number: P14136), and neurofilament light polypeptide (NF-L, Uniprot accession number: P07196) have low detection accuracy when each is used alone as a biomarker, but Tau, NF-L, and GFAP tend to increase in patients with a neurodegenerative disease more than in healthy subjects, and α-synuclein and Aβ-42 tend to decrease in patients with a neurodegenerative disease more than in healthy subjects.

One example of the model to distinguish a subject from a neurodegenerative disease patient or neurodegenerative disease patients from each other is represented by Equation (2) below:

$$\text{SCORE} = (a + Z_{FABP3})(a + Z_{Tau})^x(a + Z_{NF\text{-}L})^y(a + Z_{GFAP})^z/\{(a + Z_{FABP5}) \times (a + Z_{a\text{-}syn})^m \times (a + Z_{A\beta\text{-}42})^n\} \times a^{(m+n-x-y-z)} \quad (2)$$

where

$Z_{FABP3}$ is (FABP3 concentration in a biological sample from a subject - FABP3 mean concentration in biological samples from healthy subjects)/(standard deviation of FABP3 concentrations in biological samples from healthy subjects),

$Z_{Tau}$ is (Tau concentration in a biological sample from a subject - Tau mean concentration in biological samples from healthy subjects)/(standard deviation of Tau concentrations in biological samples from healthy subjects),

$Z_{NF\text{-}L}$ is (NF-L concentration in a biological sample from a subject - NF-L mean concentration in biological samples from healthy subjects)/(standard deviation of NF-L concentrations in biological samples from healthy subjects),

$Z_{GFAP}$ is (GFAP concentration in a biological sample from a subject - GFAP mean concentration in biological samples from healthy subjects)/(standard deviation of GFAP concentrations in biological samples from healthy subjects),

$Z_{FABP5}$ is (FABP5 concentration in a biological sample from a subject - FABP5 mean concentration in biological samples from healthy subjects)/(standard deviation of FABP5 concentrations in biological samples from healthy subjects),

$Z_{\alpha\text{-}syn}$ is (α-synuclein concentration in a biological sample from a subject - α-synuclein mean concentration in biological samples from healthy subjects)/(standard deviation of α-synuclein concentrations in biological samples from healthy subjects),

$Z_{A\beta\text{-}42}$ is (Aβ-42 concentration in a biological sample from a subject - Aβ-42 mean concentration in biological samples from healthy subjects)/(standard deviation of Aβ-42 concentrations in biological samples from healthy subjects),

a is a number greater than zero, that is, a positive number, preferably a positive integer or a positive decimal, and more preferably a number ranging from 1 to 10, and for example, a can be any integer from 1 to 10, and

x, y, z, m, and n are independently 1 when evaluation values of biomarkers Tau, GFAP, α-synuclein, Aβ-42, and NF-L associated at bases of these multipliers in Equation (2) are calculated, or 0 when the evaluation values are not calculated.

The above SCORE is used for the evaluation value or reference value, the index to detect a neurodegenerative disease.

In Equation (2), the SCORE value is 1 in healthy subjects, but the mean value of SCORE is greater than 1 in all patients with mild cognitive impairment, Alzheimer's disease, Parkinson's disease, or dementia with Lewy bodies.

In some embodiments, in the method for detecting a neurodegenerative disease in a subject of the present invention, levels of four biomarkers FABP3, FABP5, α-synuclein, and Aβ-42 in a biological sample from a subject are measured. When the levels of the four biomarkers FABP3, FABP5, α-synuclein, and Aβ-42 are substituted into Equation (2), SCORE values are significantly different respectively between the mean value of a healthy subject group and the mean value of a patient group with mild cognitive impairment, between the mean value of a healthy subject group and the mean value of a patient group with Alzheimer's disease, between the mean values of healthy subjects and a patient group with Parkinson's disease, between the mean value of a healthy subject group and the mean value of a patient group with Lewy body disease, between the mean value of a patient group with mild cognitive impairment and the mean value of a patient group with Lewy body disease, between the mean value of a patient group with Alzheimer's disease and the mean value of a patient group with Parkinson's disease, between the mean value of a patient group with Alzheimer's disease and the mean value of a patient group with Lewy body disease, and between the mean value of a patient group with Parkinson's disease and the mean value of a patient group with Lewy body disease, and the latter is significantly greater than the former, respectively.

In some embodiments, in the method for detecting a neurodegenerative disease in a subject of the present invention, levels of five biomarkers FABP3, FABP5, α-synuclein, Aβ-42, and GFAP in a biological sample from a subject are measured. When the levels of the five biomarkers FABP3, FABP5, α-synuclein, Aβ-42, and GFAP are substituted into Equation (2), SCORE values are significantly different respectively between the mean value of a healthy subject group and the mean value of a patient group with mild cognitive impairment, between the mean value of a healthy subject group and the mean value of a patient group with Alzheimer's disease, between the mean value of a healthy subject group and the mean value of a Parkinson's disease patient group, between the mean value of a healthy subject group and the mean value of a patient group with Lewy body disease, between the mean value of a patient group with mild cognitive impairment and the mean value of a patient group with Alzheimer's disease, between the mean value of a patient group with mild cognitive impairment and the mean value of a patient group with Lewy body disease, between the mean value of a patient group with Alzheimer's disease and the mean value of a Parkinson's disease patient group, and between the mean value of a Parkinson's disease patient group and the mean value of a patient group with Lewy body disease, and the latter is significantly greater than the former, respectively.

In some embodiments, in the method for detecting a neurodegenerative disease in a subject of the present invention, levels of six biomarkers FABP3, FABP5, α-synuclein, Aβ-42, GFAP, and NF-L in a biological sample from a subject are measured. When the levels of the six biomarkers FABP3, FABP5, α-synuclein, Aβ-42, GFAP, and NF-L are substituted into Equation (2), SCORE values are significantly different respectively between the mean value of a healthy subject group and the mean value of a patient group with mild cognitive impairment, between the mean value of a healthy subject group and the mean value of a patient group with Alzheimer's disease, between the mean values of healthy subjects and a Parkinson's disease patient group, between the mean value of a healthy subject group and the mean value of Lewy body disease, between the mean value of a patient group with mild cognitive impairment and the mean value of a patient group with Alzheimer's disease, between the mean value of a patient group with mild cognitive impairment and the mean value of a patient group with Lewy body disease, between the mean value of a patient group with Alzheimer's disease and the mean value of a Parkinson's disease patient group, and between the mean value of a Parkinson's disease patient group and the mean value of a patient group with Lewy body disease, and the latter is significantly greater than the former, respectively.

Combining thus one or two or more of further biomarkers Tau, GFAP, α-synuclein, Aβ-42, and NF-L with FABP3 and FABP5 can individually distinguish four types of neurodegenerative diseases, mild cognitive impairment (MCI), Alzheimer's disease (AD), Parkinson's disease (PD), and dementia with Lewy bodies (DLB).

Thus, in the detection method of the present invention, the likelihood of having a neurodegenerative disease including mild cognitive impairment is first examined or determined using FABP3 or FABP5 in a first step, and when the result of the first step is positive, a level(s) of one or two or more additional biomarkers Tau, GFAP, α-synuclein, Aβ-42, and NF-L are examined, in addition to FABP3 and FABP5, and this can individually distinguish four types, mild cognitive impairment (MCI), Alzheimer's disease (AD), Parkinson's disease (PD), and dementia with Lewy bodies (DLB), and can examine or determine the likelihood of progression from mild cognitive impairment (MCI) to Alzheimer's disease (AD), Parkinson's disease (PD), or dementia with Lewy bodies (DLB).

The detection method of the present invention is applicable to detection or diagnosis of a neurodegenerative disease or assistance for detection or diagnosis of a neurodegenerative disease instead of a diagnostic method for a neurodegenerative disease in the art or in combination with a diagnostic method for a neurodegenerative disease in the art.

The detection method of the present invention can also be performed by collecting biological samples from a patient in chronological order and examining change over time in the expression of the peptide of the present invention in each sample. The sampling interval of the biological samples is not particularly limited, but the biological samples are desirably sampled as frequently as possible in a range that does not impair the quality of life (QOL) of a patient. For example, when plasma or serum is used as a sample, blood is preferably collected for a period of about one day to about one year.

Furthermore, the method for detecting a neurodegenerative disease by the chronological sampling can be used to evaluate therapeutic effect of treatment when a therapeutic measure for the disease is taken for the patient who is a subject between the sampling before and the latest sampling. That is, for samples collected before and after the treatment, when decrease or increase in FABP3 and FABP5 as well as optional another or other biomarker proteins (improvement of a pathological condition) is determined to be observed in the state after the treatment compared with the state before the treatment, an evaluation can be made that the treatment has been effective. On the other hand, when decrease or increase in FABP3 and FABP5 as well as optional another or other biomarker proteins (improvement of a pathological condition) is determined not to be observed or determined to be further aggravated in the state after the treatment compared with the state before the treatment, an evaluation can be made that the treatment has not been effective.

Furthermore, the examination method for detecting a neurodegenerative disease by chronological sampling can be used to evaluate the preventive effect after a measure to reduce the risk of having a neurodegenerative disease, such as consumption of health food or the like, smoking cessation, exercise therapy, and isolation from a harmful environment. That is, for samples collected before and after taking the measure to reduce the risk of having a neurodegenerative disease, when decrease or increase in FABP3 and FABP5 as well as optional another or other biomarker proteins (onset and progression of a pathological condition) is determined not to be observed in the state after taking the measure compared with the state before taking the measure, an evaluation can be made that taking the measure has been effective. On the other hand, when increase or decrease in FABP3 and FABP5 as well as optional another or other biomarker proteins (improvement of a pathological condition) is determined not to be observed or the pathological condition is determined to be further aggravated in the state after the treatment compared with the state before the treatment, an evaluation can be made that taking the measure has not been effective.

Thus, FABP3 and FABP5 as well as other optional biomarker proteins, and the method of the present invention can be not only markers for diagnosing or detecting a neurodegenerative disease but also markers for predicting the prognosis of a neurodegenerative disease and markers for determining therapeutic effect. That is, FABP3 and FABP5 as well as other optional biomarker proteins, and the method of the present invention can be used to screen a drug discovery target molecule for treatment of a neurodegenerative disease and/or can be used as companion diagnostics to select a patient (responder) or to adjust a dosage (dose) of a therapeutic drug.

Notably, as shown in Examples described later, the present invention enables the selection of a therapeutic drug and a responder in a very early period after administration of a therapeutic drug, which has been impossible by current cognitive function tests.

In addition, FABP3 and FABP5 as well as optional other biomarker proteins and the method of the present invention can be used in methods for screening substances. The substances in this case include food products, such as health foods and food for specified health use products, that prevent a neurodegenerative disease in the presymptomatic disease stage, markers that diagnose or detect a neurodegenerative disease, and pharmaceuticals, such as therapeutic drugs, that treat a neurodegenerative disease after having the disease.

The present invention includes a method for detecting or determining a neurodegenerative disease using antibodies against FABP3 protein and FABP5 protein. Such a method is particularly useful in that it can detect the biomarker proteins with high sensitivity and high accuracy without using special equipment, such as the mass spectrometer described above, if an optimized immunoassay system is constructed and made into a kit. Specifically, examination using FABP3 and FABP5 reveals the likelihood of MCI, AD, PD, or DLB and a presumable disease can be presumed. The identification of the disease is then expected to be attempted by examination using FABP3 and FABP5 as well as one or two or more of biomarkers Tau, GFAP, α-synuclein, Aβ-42, and NF-L.

In some embodiments, a set of detection or determination agents containing antibodies against each of FABP3, FABP5, α-synuclein, and Aβ-42 is provided, the set of detection or determination agents to classify a subject as either of two options in any of the following (1) to (8): (1) a healthy subject or a patient with mild cognitive impairment, (2) a healthy subject or a patient with Alzheimer's disease, (3) a healthy subject or a patient with Parkinson's disease, (4) a healthy subject or a patient with Lewy body disease, (5) a patient with mild cognitive impairment or a patient with Lewy body disease, (6) a patient with Alzheimer's disease or a patient with Parkinson's disease, (7) a patient with Alzheimer's disease or a patient with Lewy body disease, or (8) a patient with Parkinson's disease or a patient with Lewy body disease.

Detection or determination using the set of detection or determination agents can be performed using a biological sample from a subject, for example, by the method for detecting a neurodegenerative disease using Formula (2).

In some embodiments, a set of detection or determination agents containing antibodies against each of FABP3, FABP5, α-synuclein, Aβ-42, and GFAP is provided, the set of detection or determination agents to classify a subject as either of two options in any of the following (1) to (8): (1) a healthy subject or a patient with mild cognitive impairment, (2) a healthy subject or a patient with Alzheimer's disease, (3) a healthy subject or a patient with Parkinson's disease, (4) a healthy subject or a patient with Lewy body disease, (5) a patient with mild cognitive impairment or a patient with Alzheimer's disease, (6) a patient with mild cognitive impairment or a patient with Lewy body disease, (7) a patient with Alzheimer's disease or a patient with Parkinson's disease, or (8) a patient with Parkinson's disease or a patient with Lewy body disease. Detection or determination using the set of detection or determination agents can be performed using a biological sample from a subject, for example, by the method for detecting a neurodegenerative disease using Formula (2).

In some embodiments, a set of detection or determination agents containing antibodies against each of FABP3, FABP5, α-synuclein, Aβ-42, GFAP, and NF-L is provided, the set of detection or determination agents to classify as either of two options in any of (1) to (8): (1) a healthy subject or a patient with mild cognitive impairment, (2) a healthy subject or a patient with Alzheimer's disease, (3) a healthy subject or a patient with Parkinson's disease, (4) a healthy subject or a patient with Lewy body disease, (5) a patient with mild cognitive impairment or a patient with Alzheimer's disease, (6) a patient with mild cognitive impairment or a patient with Lewy body disease, (7) a patient with Alzheimer's disease or a patient with Parkinson's disease, or (8) a patient with Parkinson's disease or a patient with Lewy body disease. Detection or determination using the set of detection or determination agents can be performed using a biological sample from a subject, for example, by the method for detecting a neurodegenerative disease using Formula (2).

In some embodiments, a detection or determination kit containing antibodies against each of FABP3, FABP5, α-synuclein, and Aβ-42 is provided, the detection or determination kit to classify a subject as either of two options in any of the following (1) to (8): (1) a healthy subject or a patient with mild cognitive impairment, (2) a healthy subject or a patient with Alzheimer's disease, (3) a healthy subject or a patient with Parkinson's disease, (4) a healthy subject or a patient with Lewy body disease, (5) a patient with mild cognitive impairment or a patient with Lewy body disease, (6) a patient with Alzheimer's disease or a patient with Parkinson's disease, (7) a patient with Alzheimer's disease or a patient with Lewy body disease, or (8) a patient with Parkinson's disease or a patient with Lewy body disease. Detection or determination using the detection or determination kit can be performed using a biological sample from a subject, for example, by the method for detecting a neurodegenerative disease using Formula (2).

In some embodiments, a detection or determination kit containing antibodies against each of FABP3, FABP5, α-synuclein, Aβ-42, and GFAP is provided, the detection or determination kit to classify a subject as either of two options in any of the following (1) to (8): (1) a healthy subject or a patient with mild cognitive impairment, (2) a healthy subject or a patient with Alzheimer's disease, (3) a healthy subject or a patient with Parkinson's disease, (4) a healthy subject or a patient with Lewy body disease, (5) a patient with mild cognitive impairment or a patient with Alzheimer's disease, (6) a patient with mild cognitive impairment or a patient with Lewy body disease, (7) a patient with Alzheimer's disease or a patient with Parkinson's disease, or (8) a patient with Parkinson's disease or a patient with Lewy body disease. Detection or determination using the detection or determination kit can be performed using a biological sample from a subject, for example, by the method for detecting a neurodegenerative disease using Formula (2).

In some embodiments, a detection or determination kit containing antibodies against each of FABP3, FABP5, α-synuclein, Aβ-42, GFAP, and NF-L is provided, the detection or determination kit to classify as either of two options in any of (1) to (8): (1) a healthy subject or a patient with mild cognitive impairment, (2) a healthy subject or a patient with Alzheimer's disease, (3) a healthy subject or a patient with Parkinson's disease, (4) a healthy subject or a patient with Lewy body disease, (5) a patient with mild cognitive impairment or a patient with Alzheimer's disease, (6) a patient with mild cognitive impairment or a patient with Lewy body disease, (7) a patient with Alzheimer's disease or a patient with Parkinson's disease, or (8) a patient with Parkinson's disease or a patient with Lewy body disease. Detection or determination using the detection or determination kit can be performed using a biological sample from a subject, for example, by the method for detecting a neurodegenerative disease using Formula (2).

The disclosures of all patent applications and literatures cited in the present specification are incorporated herein by reference in their entirety.

Hereinafter, the present invention will be described more specifically with reference to examples, but the present invention is not limited to these.

EXAMPLES

1. Subjects and Blood Collection

At the National Hospital Organization Sendai Nishitaga Hospital, 3 mL of blood was collected from each of 30 healthy subjects, 110 MCI patients, 146 AD patients, 85 PD patients, and 46 DLB patients. The collected blood was allowed to stand for 0.5 to 1.0 hr and then centrifuged at 3000 rpm for 10 minutes at room temperature, and serum was obtained. The supernatant was stored in aliquots at -80°C until use.

The National Institute on Aging-Alzheimer's Association (NIA/AA) was adopted as the diagnostic criteria for MCI. The National Institute of Neurologic, Communicative Disorders and Stroke AD and Related Disorders Association (NINCDS-ADRDA) was adopted as the diagnostic criteria for AD. The Movement Disorders Society was adopted as the diagnostic criteria for PD, and the El Escorial criteria was adopted as the diagnostic criteria for ALS patients. In addition, the Diagnostic and Statistical Manual of Mental Disorders, Fifth Edition (DSM-5) was adopted for the diagnostic criteria for DLB.

2. Analysis Using Antibody

An Ultra-Sensitive Auto ELISA Simoa HD-X System (Quanterix) was used to measure each biomarker in plasma. First, surfaces of Simoa magnetic beads were coated with a capture antibody, and one antibody for detection was biotinylated. These capture antibodies and detection antibodies were then used to construct calibration curves using each standard protein, and biomarker concentrations in plasma were calculated. In addition, a plasma sample was vortexed, then the supernatant was centrifuged at 14000 x g, diluted 5- to 20-fold, and measured.

3. Statistical Analysis

Prism software was used for statistical analysis. ROC analysis was performed to evaluate the diagnostic ability of the constructed model. An R package "Epi package" (a package for statistical analysis in epidemiology, Version 1.149, http://cran.r-project.org/web/packages/Epi/index.html) was used. The AUC was calculated from the ROC curve. Optimal cutoff values for diagnosis were determined according to Youden's index in Cancer 1950; 3: 32-5.

4. Identification of Biomarker Proteins

An Ultra-Sensitive Auto ELISA Simoa HD-X System (Quanterix) was used to measure each biomarker in plasma.

5. Diagnostic Performances of Biomarker Proteins in Various Neurodegenerative Diseases

Prism software was used to construct ROC curves and calculate AUC values.

6. Results

First, concentration of only one biomarker protein FABP3 in plasma was measured and statistical analysis was performed.

As a result, as shown in FIGS. 1 (A) to (D), no significant difference was found for plasma FABP3 concentration between the healthy control group and each group of mild cognitive impairment, Alzheimer's disease, Parkinson's disease, and dementia with Lewy bodies.

FIGS. 2 (A) to (D) and Table 1 also failed to distinguish between the healthy subjects and each of the patient group with mild cognitive impairment, the patient group with Alzheimer's disease, the patient group with Parkinson's disease, and the patient group with Lewy body disease.

[Table 1]

| TABLE 1. Diagnostic Performances Using FABP3 | | | |
|---|---|---|---|
| | Standard error | P value | Significant difference |
| CN vs MCI | -2.726 | 0.1870 | None |
| CN vs AD | -2.039 | 0.5029 | None |
| CN vs PD | -2.907 | 0.1815 | None |
| CN vs DLB | -3.450 | 0.1249 | None |
| MCI vs AD | 0.6872 | 0.9052 | None |
| MCI vs PD | -0.1811 | 0.9995 | None |
| MCI vs DLB | -0.7236 | 0.9578 | None |

(continued)

| TABLE 1. Diagnostic Performances Using FABP3 | | | |
|---|---|---|---|
| | Standard error | P value | Significant difference |
| AD vs PD | -0.8683 | 0.8693 | None |
| AD vs DLB | -1.411 | 0.6895 | None |
| PD vs DLB | -0.5425 | 0.9892 | None |
| *Turkey's multiple comparisons test | | | |

Then, antibodies against two biomarker proteins FABP3 and FABP5 were used, and statistical analysis was performed using the model represented by Equation (1).

As a result, as shown in FIGS. 3 (A) to (D), significant differences were found between a SCORE value of the healthy control group (SCORE = 1) and SCORE values of each group (SCORE > 1) of mild cognitive impairment, Alzheimer's disease, Parkinson's disease, and dementia with Lewy bodies, and this enabled the healthy control group to be distinguished from the four neurodegenerative disease groups.

In addition, FIGS. 4 (A) to (I) and Table 2 show that the groups were able to be distinguished from each other in the following each combination: (1) the healthy subject group and the patient group with mild cognitive impairment, (2) the healthy subject group and the patient group with Alzheimer's disease, (3) the healthy subject group and the patient group with Parkinson's disease, (4) the healthy subject group and the patient group with Lewy body disease, (5) the patient group with mild cognitive impairment and the patient group with Alzheimer's disease, (6) the patient group with mild cognitive impairment and the patient group with Parkinson's disease, (7) the patient group with mild cognitive impairment and the patient group with Lewy body disease, (8) the patient group with Alzheimer's disease and the patient group with Parkinson's disease, and (9) the patient group with Parkinson's disease and the patient group with Lewy body disease.

[Table 2]

| TABLE 2. Diagnostic Performances Using FABP3 and FABP5 | | | |
|---|---|---|---|
| | Area under curve | Standard error | P value | Diagnosis |
| CN vs MCI | 0.8255 | 0.04137 | <0.0001 | excellent |
| CN vs AD | 0.8849 | 0.03377 | <0.0001 | excellent |
| CN vs PD | 0.6843 | 0.05110 | 0.0028 | good |
| CN vs DLB | 0.8738 | 0.04117 | <0.0001 | excellent |
| MCI vs AD | 0.5872 | 0.03611 | 0.0167 | possible |
| MCI vs PD | 0.6030 | 0.04178 | 0.0136 | possible |
| MCI vs DLB | 0.6136 | 0.05051 | 0.0242 | possible |
| AD vs PD | 0.6780 | 0.03834 | <0.0001 | excellent |
| PD vs DLB | 0.6954 | 0.04685 | 0.0002 | good |
| $p < 0.0001$ excellent; $p < 0.001$, $p < 0.01$ good; $p < 0.05$ possible (Wilson-Brown) | | | |

Then, antibodies against each of four biomarkers FABP3, FABP5, $\alpha$-synuclein, and A$\beta$-42 were used, and statistical analysis was performed using the model represented by Equation (2).

As a result, as shown in FIGS. 5 (A) to (D), significant differences were found between a SCORE value of the healthy control group (SCORE = 1) and SCORE values of each group (SCORE > 1) of mild cognitive impairment, Alzheimer's disease, Parkinson's disease, and dementia with Lewy bodies, and this enabled the healthy control group to be distinguished from the four neurodegenerative disease groups.

In addition, FIGS. 6 (A) to (I) and Table 3 show that the groups were able to be distinguished from each other in the following each combination: (1) the healthy subject group and the patient group with mild cognitive impairment, (2) the healthy subject group and the patient group with Alzheimer's disease, (3) the healthy subject group and the patient group with Parkinson's disease, (4) the healthy subject group and the patient group with Lewy body disease, (5) the patient group with mild cognitive impairment and the patient group with Lewy body disease, (6) the patient group with Alzheimer's disease and the patient group with Parkinson's disease, (7) the patient group with Alzheimer's disease and the patient group with Lewy body disease, and (8) the patient group with Parkinson's disease and the patient group with Lewy body disease.

[Table 3]

| TABLE 3. Diagnostic Performances Using FABP3, FABP5, $\alpha$-Synuclein, and A$\beta$-42 | | | | |
|---|---|---|---|---|
| | Area under curve | Standard error | P value | Diagnosis |
| CN vs MCI | 0.8752 | 0.03104 | <0.0001 | excellent |
| CN vs AD | 0.9310 | 0.02348 | <0.0001 | excellent |
| CN vs PD | 0.8102 | 0.04006 | <0.0001 | excellent |
| CN vs DLB | 0.9433 | 0.03041 | <0.0001 | excellent |
| MCI vs DLB | 0.7457 | 0.04541 | <0.0001 | excellent |
| AD vs PD | 0.6315 | 0.04133 | 0.0020 | good |
| AD vs DLB | 0.7011 | 0.04908 | <0.0001 | excellent |
| PD vs DLB | 0.7702 | 0.04405 | <0.0001 | excellent |
| *p* < 0.0001 excellent; *p* < 0.001, *p* < 0.01 good; *p* < 0.05 possible (Wilson-Brown) | | | | |

Then, antibodies against each of five biomarkers FABP3, FABP5, $\alpha$-synuclein, A$\beta$-42, and GFAP were used, and statistical analysis was performed using the model represented by Equation (2).

As a result, as shown in FIGS. 7 (A) to (D), significant differences were found between a SCORE value of the healthy control group (SCORE = 1) and SCORE values of each group (SCORE > 1) of mild cognitive impairment, Alzheimer's disease, Parkinson's disease, and dementia with Lewy bodies, and this enabled the healthy control group to be distinguished from the four neurodegenerative disease groups.

In addition, FIGS. 8 (A) to (I) and Table 4 show that the groups were able to be distinguished from each other in the following each combination: (1) the healthy subject group and the patient group with mild cognitive impairment, (2) the healthy subject group and the patient group with Alzheimer's disease, (3) the healthy subject group and the patient group with Parkinson's disease, (4) the healthy subject group and the patient group with Lewy body disease, (5) the patient group with mild cognitive impairment and the patient group with Alzheimer's disease, (6) the patient group with mild cognitive impairment and the patient group with Lewy body disease, (7) the patient group with Alzheimer's disease and the patient group with Parkinson's disease, and (8) the patient group with Parkinson's disease and the patient group with Lewy body disease.

[Table 4]

| TABLE 4. Diagnostic Performances Using FABP3, FABP5, $\alpha$-Synuclein, A$\beta$-42, and GFAP | | | | |
|---|---|---|---|---|
| | Area under curve | Standard error | P value | Diagnosis |
| CN vs MCI | 0.8887 | 0.03190 | <0.0001 | excellent |
| CN vs AD | 0.9892 | 0.007927 | <0.0001 | excellent |
| CN vs PD | 0.8416 | 0.03932 | <0.0001 | excellent |
| CN vs DLB | 0.9511 | 0.02902 | <0.0001 | excellent |
| MCI vs AD | 0.8028 | 0.03030 | <0.0001 | excellent |
| MCI vs DLB | 0.7732 | 0.04169 | <0.0001 | excellent |
| AD vs PD | 0.8569 | 0.02728 | <0.0001 | excellent |
| PD vs DLB | 0.8280 | 0.03933 | <0.0001 | excellent |
| *p* < 0.0001 excellent; *p* < 0.001, *p* < 0.01 good; *p* < 0.05 possible (Wilson-Brown) | | | | |

Then, antibodies against each of six biomarkers FABP3, FABP5, $\alpha$-synuclein, A$\beta$-42, GFAP, and NF-L were used, and statistical analysis was performed using the model represented by Equation (2).

As a result, as shown in FIGS. 9 (A) to (D), significant differences were found between a SCORE value of the healthy control group (SCORE = 1) and SCORE values of each group (SCORE > 1) of mild cognitive impairment, Alzheimer's disease, Parkinson's disease, and dementia with Lewy bodies, and this enabled the healthy control group to be distinguished from the four neurodegenerative disease groups.

In addition, FIGS. 10 (A) to (I) and Table 5 show that the groups were able to be distinguished from each other in the following each combination: (1) the healthy subject group and the patient group with mild cognitive impairment, (2) the healthy subject group and the patient group with Alzheimer's disease, (3) the healthy subject group and the patient group with Parkinson's disease, (4) the healthy subject group and the patient group with Lewy body disease, (5) the patient group with mild cognitive impairment and the patient group with Alzheimer's disease, (6) the patient group with mild cognitive impairment and the patient group with Lewy body disease, (7) the patient group with Alzheimer's disease and the patient group with Parkinson's disease, and (8) the patient group with Parkinson's disease and the patient group with Lewy body disease.

[Table 5] TABLE 5. Diagnostic Performances Using FABP3, FABP5, $\alpha$-Synuclein, A$\beta$-42, GFAP, and NF-L

|  | Area under curve | Standard error | P value | Diagnosis |
|---|---|---|---|---|
| CN vs MCI | 0.8928 | 0.03008 | <0.0001 | excellent |
| CN vs AD | 0.9918 | 0.005123 | <0.0001 | excellent |
| CN vs PD | 0.9012 | 0.02937 | <0.0001 | excellent |
| CN vs DLB | 0.9865 | 0.009664 | <0.0001 | excellent |
| MCI vs AD | 0.7958 | 0.03065 | <0.0001 | excellent |
| MCI vs DLB | 0.8145 | 0.03676 | <0.0001 | excellent |
| AD vs PD | 0.7903 | 0.03336 | <0.0001 | excellent |
| PD vs DLB | 0.8098 | 0.03958 | <0.0001 | excellent |
| $p < 0.0001$ excellent; $p < 0.001$, $p < 0.01$ good; $p < 0.05$ possible | | | | |

The above results suggested that the predictive value obtained by the multivariate regression model from the blood concentrations of these biomarkers will be useful for an examination method for determination or early diagnosis of a neurodegenerative disease.

**Claims**

1. A method for detecting a neurodegenerative disease in a subject, the method comprising:
   measuring levels of two biomarkers comprising FABP3 and FABP5 in a biological sample from a subject.

2. The method according to claim 1, the method further comprising:

   applying and/or converting levels of the two biomarkers comprising FABP3 and FABP5 to a model to produce an evaluation value that is an index to detect a neurodegenerative disease; and
   determining whether a subject is likely to have a neurodegenerative disease by comparing the evaluation value in the subject with a reference value.

3. The method according to claim 2, wherein the determining comprises determining that the subject is likely to have a neurodegenerative disease when the evaluation value of the subject is higher than an evaluation value of a healthy subject.

4. The method according to claim 3, wherein when the subject is determined to be likely to have a mild cognitive impairment, Alzheimer's disease, Parkinson's disease, or dementia with Lewy bodies, the method further comprises:

   comparing the evaluation value of the subject with each of a reference value that is an evaluation value of a patient with mild cognitive impairment, a reference value that is an evaluation value of a patient with Alzheimer's disease, a reference value that is an evaluation value of a patient with Parkinson's disease, and a reference value that is an evaluation value of a patient with dementia with Lewy bodies; and
determining that the subject is likely to have a disease with the smallest numerical difference between the evaluation value of the subject and each of the reference values of the four diseases.

5. The method according to any one of claims 2 to 4, wherein the model is

$$\mathrm{SCORE} = (a + Z_{\mathrm{FABP3}})/(a + Z_{\mathrm{FABP5}}) \quad (1)$$

where

$Z_{\mathrm{FABP3}}$ is (FABP3 concentration in a biological sample from a subject - FABP3 mean concentration in biological samples from healthy subjects)/(standard deviation of FABP3 concentrations in biological samples from healthy subjects),
$Z_{\mathrm{FABP5}}$ is (FABP5 concentration in a biological sample from a subject - FABP5 mean concentration in biological samples from healthy subjects)/(standard deviation of FABP5 concentrations in biological samples from healthy subjects), and
a is a number greater than zero; and

the SCORE is used for the evaluation value or the reference value.

6. The method according to claim 1, comprising further measuring a level of at least one or more biomarkers selected from the group consisting of Tau, $\alpha$-synuclein, A$\beta$-42, GFAP, and NF-L in a biological sample from a subject.

7. The method according to claim 6, further comprising:

applying and/or converting a level of at least one or more further biomarkers selected from the group consisting of Tau, $\alpha$-synuclein, A$\beta$-42, GFAP, and NF-L in addition to the two biomarkers FABP3 and FABP5 to a model to produce an evaluation value that is an index to detect a neurodegenerative disease; and
determining whether a subject is likely to have a neurodegenerative disease by comparing an evaluation value of the subject with a reference value that is an evaluation value of a neurodegenerative disease patient.

8. The method according to claim 7, wherein the neurodegenerative disease is at least one selected from the group consisting of mild cognitive impairment, Alzheimer's disease, Parkinson's disease, and dementia with Lewy bodies, and
the determining comprises:

determining that the subject is likely to have mild cognitive impairment when the evaluation value of the subject is equal to or higher than a reference value that is an evaluation value of a patient with mild cognitive impairment;
determining that the subject is likely to have Alzheimer's disease when the evaluation value of the subject is equal to or higher than a reference value that is an evaluation value of a patient with Alzheimer's disease;
determining that the subject is likely to have Parkinson's disease when the evaluation value of the subject is equal to or higher than a reference value that is an evaluation value of a patient with Parkinson's disease; or
determining that the subject is likely to have dementia with Lewy bodies when the evaluation value of the subject is equal to or higher than a reference value that is an evaluation value of a patient with dementia with Lewy bodies.

9. The method according to claim 8, wherein when the subject is determined to be likely to have mild cognitive impairment, Alzheimer's disease, Parkinson's disease, or dementia with Lewy bodies described above, the method further comprises:

comparing the evaluation value of the subject with each of a reference value that is an evaluation value of a patient with mild cognitive impairment, a reference value that is an evaluation value of a patient with Alzheimer's disease, a reference value that is an evaluation value of a patient with Parkinson's disease, and a reference value that is an evaluation value of a patient with dementia with Lewy bodies; and
determining that the subject is likely to have a disease with the smallest numerical difference between the evaluation value of the subject and each of the reference values of the four diseases.

10. The method according to any one of claims 7 to 9, wherein the model is

$$\text{SCORE} = (a + Z_{FABP3})(a + Z_{Tau})^x(a + Z_{NF\text{-}L})^y(a + Z_{GFAP})^z/\{(a + Z_{FABP5}) \times (a + Z_{a\text{-}syn})^m \times (a + Z_{A\beta\text{-}42})^n\} \times a^{(m+n-x-y-z)} \quad (2)$$

where

$Z_{FABP3}$ is (FABP3 concentration in a biological sample from a subject - FABP3 mean concentration in biological samples from healthy subjects)/(standard deviation of FABP3 concentration in biological samples from healthy subjects),

$Z_{Tau}$ is (Tau concentration in a biological sample from a subject - Tau mean concentration in biological samples from healthy subjects)/(standard deviation of Tau concentrations in biological samples from healthy subjects),

$Z_{NF\text{-}L}$ is (NF-L concentration in a biological sample from a subject - NF-L mean concentration in biological samples from healthy subjects)/(standard deviation of NF-L concentrations in biological samples from healthy subjects),

$Z_{GFAP}$ is (GFAP concentration in a biological sample from a subject - GFAP mean concentration in biological samples from healthy subjects)/(standard deviation of GFAP concentrations in biological samples from healthy subjects),

$Z_{FABP5}$ is (FABP5 concentration in a biological sample from a subject - FABP5 mean concentration in biological samples from healthy subjects)/(standard deviation of FABP5 concentrations in biological samples from healthy subjects),

$Z_{\alpha\text{-}syn}$ is ($\alpha$-synuclein concentration in a biological sample from a subject - $\alpha$-synuclein mean concentration in biological samples from healthy subjects)/(standard deviation of $\alpha$-synuclein concentrations in biological samples from healthy subjects),

$Z_{A\beta\text{-}42}$ is (A$\beta$-42 concentration in a biological sample from a subject - A$\beta$-42 mean concentration in biological samples from healthy subjects)/(standard deviation of A$\beta$-42 concentrations in biological samples from healthy subjects),

a is a number greater than zero, and

x, y, z, m, and n are 1 independently when evaluation values of biomarkers Tau, GFAP, $\alpha$-synuclein, A$\beta$-42, and NF-L associated at bases of these multipliers in Equation (2) are calculated, or 0 when the evaluation values are not calculated; and

the SCORE is used for the evaluation value or the reference value.

**11.** Use of a combination of two comprising FABP3 and FABP5;

a combination of four comprising FABP3, FABP5, Tau, and NF-L;
a combination of four comprising FABP3, FABP5, $\alpha$-synuclein, and A$\beta$-42;
a combination of five comprising FABP3, FABP5, $\alpha$-synuclein, A$\beta$-42, and GFAP; or
a combination of six comprising FABP3, FABP5, $\alpha$-synuclein, A$\beta$-42, GFAP, and NF-L as biomarkers to examine at least one neurodegenerative disease selected from the group consisting of mild cognitive impairment, Alzheimer's disease, Parkinson's disease, and dementia with Lewy bodies.

**12.** A kit for examination to examine at least one neurodegenerative disease selected from the group consisting of mild cognitive impairment, Alzheimer's disease, Parkinson's disease, and dementia with Lewy bodies, the kit comprising:

antibodies against each of two proteins comprising FABP3 and FABP5;
antibodies against each of four proteins comprising FABP3, FABP5, Tau, and NF-L;
antibodies against each of four proteins comprising FABP3, FABP5, $\alpha$-synuclein, and A$\beta$-42;
antibodies against each of five proteins comprising FABP3, FABP5, $\alpha$-synuclein, A$\beta$-42, and GFAP; or
antibodies against each of six proteins comprising FABP3, FABP5, $\alpha$-synuclein, A$\beta$-42, GFAP, and NF-L.

FIG. 1

FIG. 2

FIG. 3

FIG. 4

FIG. 5

FIG. 6

FIG. 7

FIG. 8

FIG. 9

FIG. 10

## INTERNATIONAL SEARCH REPORT

| International application No. |
| --- |
| **PCT/JP2022/003350** |

| | |
| --- | --- |
| **A.** | **CLASSIFICATION OF SUBJECT MATTER** |

*G01N 33/53*(2006.01)i; *C07K 16/18*(2006.01)i
FI:   G01N33/53 D; C07K16/18

According to International Patent Classification (IPC) or to both national classification and IPC

| | |
| --- | --- |
| **B.** | **FIELDS SEARCHED** |

Minimum documentation searched (classification system followed by classification symbols)

G01N33/53; C07K16/18

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN); 医中誌WEB (Ichushi WEB)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| X | COLOGNA, S. M. et al. Quantitative Proteomic Analysis of Niemann-Pick Disease, Type C1 Cerebellum Identifies Protein Biomarkers and Provides Pathological Insight. PLOS ONE. October 2012, vol. 7, no. 10, pages 1-13 <br> particularly, "abstract", "Introduction", "Fatty Acid Binding Proteins", "Discussion", fig. 6-7 | 1-3, 6-7 |
| Y | JP 2007-506086 A (ELECTROPHORETICS LTD.) 15 March 2007 (2007-03-15) <br> claims, paragraph [0061] | 1-3, 6-8, 11-12 |
| Y | DAVID, C. et al. Differential role of CSF fatty acid binding protein 3, α-synuclein, and Alzheimer's disease core biomarkers in Lewy body disorders and Alzheimer's dementia. Alzheimer's Research & Therapy. 2017, vol. 9, no. 52, pages 1-12 <br> particularly, "abstract" | 1-3, 6-8, 11-12 |
| Y | JP 2014-071016 A (MITSUBISHI CHEMICAL MEDIENCE CORP.) 21 April 2014 (2014-04-21) <br> claims, paragraph [0053] | 1-3, 6-8, 11-12 |
| Y | JP 2015-004664 A (MAGQU CO., LTD.) 08 January 2015 (2015-01-08) <br> claims | 6-8 |

☑ Further documents are listed in the continuation of Box C.　　☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **01 March 2022** | **15 March 2022** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**

International application No.

**PCT/JP2022/003350**

### C.    DOCUMENTS CONSIDERED TO BE RELEVANT

| Category* | Citation of document, with indication, where appropriate, of the relevant passages | Relevant to claim No. |
|---|---|---|
| A | WO 2014/065323 A1 (RIKEN) 01 May 2014 (2014-05-01)<br>entire text, all drawings | 1-12 |
| A | JP 2013-092538 A (UNIV. DE GENEVE) 16 May 2013 (2013-05-16)<br>entire text, all drawings | 1-12 |
| A | WO 2017/171053 A1 (TOHOKU UNIV.) 05 October 2017 (2017-10-05)<br>entire text, all drawings | 1-12 |
| A | 古橋眞人, 脂肪酸結合タンパクファミリー, 肥満研究, 2013, vol. 19, no. 3, pages 167-174, (FURUHASHI, Masato. Fatty Acid-Binding Protein Family. Journal of Japan Society for the Study of Obesity.)<br>entire text, all drawings | 1-12 |
| A | MAEKAWA, M. et al. Polymorphism screening of brain-expressed FABP7, 5 and 3 genes and association studies in autism and schizopherenia in Japanese subjects. Journal of human genetics. 08 January 2010, vol. 55, pages 127-130<br>entire text, all drawings | 1-12 |
| A | WANG, Y. et al. Epidermal Fatty Acid-Binding Protein 5 (FABP5) Involvement in Alpha-Synuclein-Induced Mitochondrial Injury under Oxidative Stress. Biomedicines. 22 January 2021, vol. 9, no. 110, pages 1-15<br>entire text, all drawings | 1-12 |
| A | PAN, Y. et al. Reduced blood-brain barrier expression of fatty acid-binding protein 5 is associated with increased vulnerability of APP/PS1 mice to cognitive deficits from low omega-3 fatty acid diets. Journal of Neurochemistry. 2018, vol. 144, pages 81-92<br>entire text, all drawings | 1-12 |
| P, A | DHIMAN, K. et al. Cerebrospinal fluid levels of fatty acid binding protein 3 associate with brain amyloidosis in cognitively healthy individuals at risk of developing Alzheimer's disease. Alzheimer's Dementia. 31 December 2021, vol. 17, suppl. 5, page 1<br>entire text | 1-12 |

Form PCT/ISA/210 (second sheet) (January 2015)

EP 4 286 848 A1

**INTERNATIONAL SEARCH REPORT**
Information on patent family members

International application No.

**PCT/JP2022/003350**

| Patent document cited in search report | | | Publication date (day/month/year) | Patent family member(s) | | | Publication date (day/month/year) |
|---|---|---|---|---|---|---|---|
| JP | 2007-506086 | A | 15 March 2007 | US | 2007/0042425 | A1 | |
| | | | | claims, paragraph [0148] | | | |
| | | | | WO | 2005/029088 | A2 | |
| | | | | EP | 2357477 | A1 | |
| JP | 2014-071016 | A | 21 April 2014 | (Family: none) | | | |
| JP | 2015-004664 | A | 08 January 2015 | US | 2014/0370518 | A1 | |
| | | | | claims | | | |
| | | | | EP | 2816353 | A1 | |
| WO | 2014/065323 | A1 | 01 May 2014 | US | 2015/0309053 | A1 | |
| | | | | entire text, all drawings | | | |
| JP | 2013-092538 | A | 16 May 2013 | US | 2008/0220013 | A1 | |
| | | | | entire text, all drawings | | | |
| | | | | WO | 2007/007129 | A2 | |
| WO | 2017/171053 | A1 | 05 October 2017 | US | 2019/0125726 | A1 | |
| | | | | entire text, all drawings | | | |
| | | | | EP | 3437640 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHIODA N et al.** *J Biol Chem.,* 04 July 2014, vol. 289 (27), 18957-65
- *The Uehara Memorial Foundation Research Report Collection,* 2017, vol. 31
- **TEUNISSEN, C.E. et al.** *European journal of neurology,* 2011, vol. 18, 865-871
- *Cancer,* 1950, vol. 3, 32-5